(19)
Europäisches
Patentamt

European
Patent Office

Office européen
des brevets

(11) **EP 2 291 111 B1**

(12) **EUROPEAN PATENT SPECIFICATION**

(45) Date of publication and mention
of the grant of the patent:
**25.04.2012 Bulletin 2012/17**

(21) Application number: **09742514.4**

(22) Date of filing: **04.05.2009**

(51) Int Cl.:
*A61B 5/1455* (2006.01)    *A61B 5/00* (2006.01)
*A61B 5/024* (2006.01)    *A61B 5/0205* (2006.01)
*A61B 5/113* (2006.01)    *A61B 5/08* (2006.01)
*G01S 13/00* (2006.01)

(86) International application number:
**PCT/IB2009/051806**

(87) International publication number:
**WO 2009/136341 (12.11.2009 Gazette 2009/46)**

(54) **CONTACTLESS RESPIRATION MONITORING OF A PATIENT AND OPTICAL SENSOR FOR A PHOTOPLETHYSMOGRAPHY MEASUREMENT**

KONTAKTLOSE ATMUNGSÜBERWACHUNG EINES PATIENTEN UND OPTISCHER SENSOR FÜR EINE PHOTOPLETHYSMOGRAPHIE-MESSUNG

SURVEILLANCE DE RESPIRATION SANS CONTACT D'UN PATIENT ET DÉTECTEUR OPTIQUE POUR UNE MESURE DE PHOTOPLÉTHYSMOGRAPHIE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.05.2008 EP 08103895**

(43) Date of publication of application:
**09.03.2011 Bulletin 2011/10**

(73) Proprietors:
• **Philips Intellectual Property & Standards GmbH 20099 Hamburg (DE)**
Designated Contracting States:
**DE**
• **Koninklijke Philips Electronics N.V. 5621 BA Eindhoven (NL)**
Designated Contracting States:
**AT BE BG CH CY CZ DK EE ES FI FR GB GR HR HU IE IS IT LI LT LU LV MC MK MT NL NO PL PT RO SE SI SK TR**

(72) Inventors:
• **PINTER, Robert**
**NL-5656 AE Eindhoven (NL)**

• **MÜHLSTEFF, Jens**
**NL-5656 AE Eindhoven (NL)**
• **SPEKOWIUS, Gerhard**
**NL-5656 AE Eindhoven (NL)**
• **YU, Donghai**
**NL-5656 AE Eindhoven (NL)**
• **DEVOT, Sandrine, M., L.**
**NL-5656 AE Eindhoven (NL)**
• **MÜSCH, Guido, J.**
**NL-5656 AE Eindhoven (NL)**
• **AUBERT, Xavier, L., M., A.**
**NL-5656 AE Eindhoven (NL)**

(74) Representative: **Van Velzen, Maaike Mathilde Philips**
**Intellectual Property & Standards**
**P.O. Box 220**
**5600 AE Eindhoven (NL)**

(56) References cited:
| | |
|---|---|
| EP-A- 0 357 249 | WO-A-97/40741 |
| WO-A-98/17174 | WO-A2-2007/143535 |
| WO-A2-2008/027509 | US-A- 5 094 240 |
| US-A1- 2005 234 312 | US-A1- 2005 288 601 |
| US-A1- 2008 077 015 | US-B1- 6 525 386 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates to the field of contactless respiration monitoring of a patient and an optical sensor for a photoplethysmography measurement, and especially to a handheld device for simultaneously monitoring respiration action, blood pressure and heart rate which can preferably used for spot-checking the vital parameters of patients in hospitals.

BACKGROUND OF THE INVENTION

It is possible to detect the pulse wave arrival in a finger of a patient by means of an optical measurement: Typically, an infrared LED shines light into the finger and the amount of light that reaches a photodiode causes a photo current to flow through the diode. In the presence of the pulse wave, a large portion of the light is absorbed by the blood, i.e. the current through the photodiode is modulated accordingly. This technique is known as photoplethysmography (PPG).

It is called "transmittive" PPG, if LED and photodiode are installed on opposite sides of the finger, such that the LED light actually shines through the finger. Such a setup is usually realised as a finger-clip. The other option is to have both LED and photodiode installed on the same side of the finger. This is called "reflective" PPG, and is useful if a finger-clip is not acceptable. In reflective mode, LED and photodiode sit next to each other, so that the patient only has to rest his finger on the two components in order to have his pulse wave detected, e.g. for heart rate measurements or pulse arrival time (PAT) measurements.

A reflective PPG setup is useful in many cases. It requires the patient only to put his finger lightly onto the LED/photodiode combination in order to have his pulse wave detected. This can be used for heart rate measurements, for example. Another application of PPG is the measurement of a pulse transit time (PTT) or of a pulse arrival time (PAT). The principle of a PTT measurement is that one takes the moment in time, when the pulse wave starts at one point of the body, and measures the arrival time at another point of the body. The PTT is calculated as the time difference between the two and is inversely related to the pulse wave velocity. The PAT is defined as the time delay between the ECG R-peak and the arrival of the PPG pulse at some peripheral site. The PPG measurements are usually done on the patient's ear lobe or on a finger.

Both PTT and PAT are interesting measures, because among other parameters, like the distance between the two measurement locations on the body and the elasticity of the blood vessels, they provide information on the blood pressure of the patient. So if the other parameters are known or can be estimated, blood pressure can be inferred from a PTT or PAT measurement.

Actually, the R peak in the ECG signal does not coincide with the start of the pressure pulse propagation in the aorta. This is because the ECG R peak is the electrical excitation of the heart muscle. It takes some time before the muscle reacts to this excitation, and then it takes even more time before the muscle has built up sufficient pressure in the heart, so that the aortic valve opens and the pulse wave really starts to travel through the arteries. However, the time delay between the R-peak and the aortic valve opening also conveys important information on the arterial blood pressure. Hence, taking the R peak as the start point for deducing the pulse wave arrival time at periphery is acceptable in many applications.

Many attempts have been made in the past to use this principle in order to provide a blood pressure measurement that does not need a cuff. Typical PAT measurement setups comprise an ECG measurement and a PPG measurement. A characteristic point of the PPG pulse is taken as the moment in time when the pulse wave arrives in the finger or ear. The difference between the occurrence time of the ECG R-peak and of the PPG characteristic point is calculated, which is translated into a blood pressure value.

The problem especially encountered in reflective PPG setups is that the pressure, with which the skin is pressed onto the LED/photodiode combination, can be so high, that the blood vessels are actually clamped off, so that the pulse wave does not reach the measurement location and therefore cannot be detected.

Further, spot-checking the vital parameters of patients in hospital beds is part of a nurse's daily routine. Heart rate, breathing frequency, blood pressure and body temperature are the most important parameters that should be checked for every patient. Measuring all these parameters properly would require a substantial effort, both in terms of measurement equipment and time. However, the practical circumstances in a hospital force the nurses to be as quick as possible with the spot-check measurements, because they have many other tasks to do, requiring more attention than the routine spot-checking.

Especially, respiratory action cannot be measured with conventional spot-checking setups yet. For that, a breathing sensor would be required. In general, such a breathing sensor had to be attached to the chest of the patient. However, attaching a sensor to the patient's chest is inconvenient and time-consuming.

US 2008/0077015A1 discloses a Doppler radar system, which may be a hand-held device, for determining physiological motion with a subject, for example heart rate and/or respiration rate of the subject. The heart rate may be compared with a reference obtained from a wired finger pressure pulse sensor.

WO 2008/027509A2 discloses a remote-detection system for monitoring changes in permittivity associated with physiological activity of a subject. Tissue of the subject is illuminated with an electromagnetic signal beam, and the reflections of the electromagnetic signal beam from the subject are detected by the system. The reflected signal includes amplitude variations indicative of motion of the illuminated tissue and amplitude variations indicative of time dependent variations in the permittivity of the illuminated tissue associated with electrical activity of the subject's heart. From the reflected signal a respiration signal structure is detected and measured next to a determination of the heart rate activity.

US 2005/0288601A1 discloses a portable handheld biofeedback device for evaluating and treating stress by identifying respiratory sinus arrhythmia (RSA) waves during respiration. The device contains a photoplethysmograph sensor and a display screen to provide subjects with near real-time information on their RSA waves. These wave patterns are used to provide a subject with near real-time respiratory feedback information based on heart rate data. Means for decreasing or adequately controlling stress levels are provided based on the wave pattern analysis and respiratory feedback.

SUMMARY OF THE INVENTION

It is an object of the invention to provide for a convenient and easy to use spot-checking possibility of the patient's respiratory action.

This object is met by a handheld device according to claim 1.

A solution for measuring respiratory action of a patient without body contact is described. It is in particular suitable for the integration into a handheld device. With respect to this the handheld device comprises a holding means which is adapted for holding the device in front of the patient's chest by the patient himself. Furthermore, it is preferred that the calculated breathing activity preferably comprises the respiration rate of the patient.

The invention provides for several advantages: Contactless measurement of respiratory action is integrated in a handheld device. Further, an easy-to-use handheld solution for doing spot-checking of heart rate, blood pressure and breathing frequency can be provided as set out in more detail in the following. Furthermore, an easy-to-use handheld solution for doing relaxation exercises, for example including breathing guidance, can be provided as set out in detail further below.

In general, different types of distance sensors like ultrasound sensors and/or laser sensors can be used. With the help of ultrasound, distance can be measured. A short ultrasound burst is transmitted towards the target, reflected at the target, and the time until the reflected burst arrives is measured. The flight time is directly proportional to the distance, because the propagation velocity is constant during the short time of measurement. Further, with the help of laser interferometry, it is possible to measure relative motion very precisely. The phase difference between emitted laser beam and reflected laser beam depends on the distance to the reflecting target, so if the reflected beam is brought to interfere with a beam that is in phase with the emitted beam, the intensity of the interference result varies periodically.

However, according to a preferred embodiment of the invention, the distance sensor is based on emitting and receiving electromagnetic waves. Further, it is preferred that the distance sensor comprises a Doppler radar sensor, preferably a two-channel Doppler radar sensor. Radar frequencies of 2.4GHz or 24GHz have shown to deliver good results.

The use of electromagnetic waves has the advantage, that they are not reflected at the clothing, but at the skin surface. Basically, reflection of electromagnetic waves occurs at boundary layers between areas of different electrical conductivity. Since the air is an electric isolator, and the clothing is usually also an isolator, there will be a reflection indeed at the surface of the skin. This is a great advantage of using electromagnetic waves.

If the reflecting target, which in this case would be the chest of the patient, is moving due to the respiratory action, the reflected electromagnetic waves are shifted in frequency with respect to the emitted waves (Doppler shift). This frequency difference can be detected and exploited as a measure for the chest motion of the patient. The principle of this measurement is known from traffic speed controls, for example. The antenna of a radar transceiver can be easily integrated in a handheld device in a way that the electromagnetic waves are directed towards the chest of the patient holding the device in his hands.

The holding means is adapted for automatically directing the distance sensor towards the patient's chest when held with both hands of the patient. In this way the handheld device is automatically aligned and no additional adjustment is necessary.

Further, the holding means comprises two handles for grabbing the device with both hands of the patient. The handles comprise electrodes for an ECG measurement. With respect to this, the handles are preferably made of metal. Furthermore, it is preferred that an ECG measuring unit is provided in the device.

According to the invention, an optical sensor for a photoplethysmography measurement, preferably an optical sensor as described below, is provided on the device. With respect to this, it is especially preferred that a reflective mode sensor is provided. Further, according to the invention, the optical sensor is positioned on the device in such a way that, when holding the device, a patient's finger, preferably a patient's thumb, automatically rests on the sensor. This makes the device more reliable also with respect to the photoplethysmography measurement. Furthermore, it is also preferred that a photoplethysmography measuring unit is provided in the device which is adapted for determining the blood pressure of the patient.

An embodiment of the invention provides a possibility for a reliable and fail-safe photoplethysmography measurement. In this embodiment the optical sensor for the photoplethysmography measurement, comprises a light unit with a light emitter for emitting light into tissue of a patient and/or a light detector for detecting a part of the emitted light after interaction with the tissue, wherein the light unit is embedded in an elastic material.

Accordingly, it is an essential idea of this embodiment to provide an elastic material which, when pressed by a patient's fingertip, is resilient and, thus, avoids clamping of capillaries in the patient's tissue. This comprises several advantages as intuitive usage of reflective finger PPG setups, and no explanation to the patient how the finger has to be applied. Further, this allows valid measurements in a reflective PPG setup irrespective of the pressure exerted on the skin that is pressed onto the light unit. Thus, this solution is simple, passive and inexpensive.

According to a preferred embodiment of the invention, the elastic material is adapted for being contacted by the patient's skin, preferably by a patient's fingertip. Further, it is preferred that the elasticity of the elastic material lies in the range of typical elasticities of the tissue of a human finger. Preferably, silicone is used for the elastic material.

In general, the invention can be applied for different types of photoplethysmography measurements. However, according to a preferred embodiment of the invention, the light unit is adapted for a reflective photoplethysmography measurement. With respect to this, according to a preferred embodiment of the invention, the light unit comprises an LED and a photodiode. Moreover, it is preferred that the elastic material is not transparent for the light emitted by the light emitter. This is advantageous since in this way, a direct light path from the light emitter to the light detector is avoided. Preferably, the feature that the elastic material is not transparent for the light emitted by the light emitter is achieved by color additives to the elastic materials.

The device described above can be used for different applications, preferably for spot-checking applications in hospitals. However, according to a preferred embodiment of the invention, an output unit is provided in the device, the output unit being adapted for outputting a stress status indicator signal, based on coherence between determined heart rate and determined breathing activity. This idea will be more apparent with the method described in the following.

A method of providing a patient with a stress status indicator signal is provided, preferably with the aid of a device as described above, comprising the following steps:

    detecting the patient's heart rate;
    simultaneously detecting the patient's breathing activity;
    calculating the degree of coherence between the heart rate and the breathing activity; and
    outputting a stress status indicator signal based on the calculated degree of coherence.

Under resting conditions, the heart rate of healthy patients exhibits a periodic variation. This rhythmic phenomenon, known as respiratory sinus arrhythmia (RSA), fluctuates with the phase of respiration: the heart rate increases during inspiration and decreases during expiration. In this way the heart rate tends to synchronize with the patient's breathing activity under certain conditions. Heart rate and respiration synchronize if a patient is in a positive or relaxed mood ("high coherence"), compared to the de-synchronization found if the patient is in a negative or stressed mood (low coherence). In the positive mood, the variation of the heart rate typically occurs in a sine wave manner. This allows to conduct simultaneously a measurement of heart rate variation and breathing activity, so the degree of coherence between the two can be calculated and used as a measure indicating the relaxation level of the patient.

According to a preferred embodiment of the invention, a guidance signal is output, indicating how the patient should breathe. Further, it is preferred that the guidance signal is automatically adapted according to the determined stress status of the patient.

Preferred applications of the invention are as follows: The invention allows contactless measurement of respiration in a handheld device. It is of particular value in a handheld device for spot-checking a patient's heart rate, blood pressure and respiratory frequency simultaneously. Furthermore, it can be used in order to build a very attractive handheld solution for giving guided breathing exercises as a technique to relax effectively from stressful situations.

BRIEF DESCRIPTION OF THE DRAWINGS

These and other aspects of the invention will be apparent from and elucidated with reference to the embodiments described hereinafter.

In the drawings:

Fig. 1 a and b schematically show a reflective PPG setup as in preferred embodiment of the invention;
Fig. 2a, b and c shows a handheld device according to the invention held by a patient;
Fig. 3 depicts a block diagram of the system according to the invention;
Fig. 4 shows how heart rate and respiration synchronize if a patient is in a positive or relaxed mood, compared to the de-synchronization found if the patient is in a negative or stressed mood;
Fig. 5 explains the calculation of coherence according to a preferred embodiment of the invention; and
Fig. 6 shows a block diagram of a system according to a preferred embodiment of the invention.

DETAILED DESCRIPTION OF EMBODIMENTS

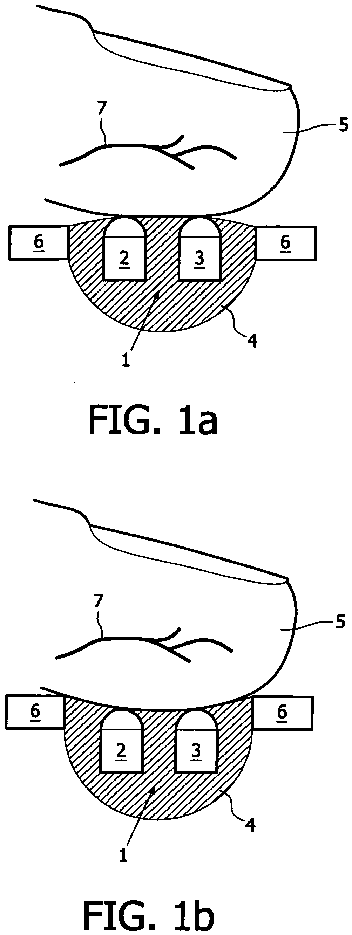

According to a preferred embodiment of the invention, it is proposed to embed the light unit 1 of an optical sensor for a reflective photoplethysmography measurement with its light emitter 2 and its light detector 3, i.e. with its LED/photodiode combination, into an elastic material 4, e.g. silicone, that will give way to the finger pressure. An according reflective PPG setup can be seen from Fig. 1. There, it is shown that a patient's finger 5 is pressed on the elastic material 4 in which the light unit 1 with the light emitter 2 and the light detector 3 are provided. On its border area, the elastic material 4 is surrounded by a rigid carrier 6. In this way clamping of the finger capillaries 7 is avoided over a wide range of finger pressures.

As can be seen from Fig. 1, the elastic material 4 is deformed depending on the amount of finger pressure applied, and because of this deformation, clamping of the capillaries 7 is avoided, thereby allowing a valid PPG measurement in this reflective PPG setup over a wide range of finger pressures. In order to achieve a wide range of tolerated finger pressures, it is preferred to choose the elasticity of the elastic material 4, in which the LED/photodiode combination is embedded, such that it is equal or similar to the elasticity of finger tissue. The elastic material 4 preferably is not transparent for the light emitted by the LED, in order to avoid a direct light path from the LED to the photodiode. This is preferably achieved with the help of color additives to the silicone, if required.

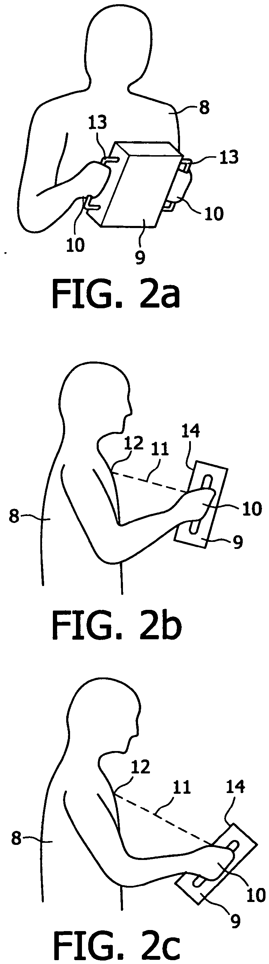

From Figs 2 a, b, and c, a handheld device 9 according to the invention can be seen. The general idea of this handheld device 9 is based on the insight that if a patient 8 holds the handheld device with both his hands 10, there is a free line of sight 11 between the handheld device 9 and the patient's chest 12 as shown in Fig. 2a, and more in detail in Figs 2b and 2c. Furthermore, the anatomy of the human arm and wrist is such that if the device has two handles 13 at the side which the patient grabs with his hands 10, the lid 14 of the handheld device 9 is automatically adjusted to point at the patient's chest 12. Figs. 2b and 2c illustrate this condition.

Since the wall of the patient's chest 12 moves forward and backward due to the respiratory action, a distance sensor is integrated into the lid 14 of the handheld device 9 that measures the distance between the lid 14 and the chest 12. Different sensor modalities are conceivable for this purpose, as described further above.

According to the preferred embodiment of the invention described here, as a distance sensor a transceiver of electromagnetic waves is provided in the handheld device 9. Experiments indicate that radar frequencies give acceptable results, preferably frequencies of 2.4GHz or 24GHz. The antenna of the radar transceiver can be easily integrated in the handheld device 9 in a way that the electromagnetic waves are directed towards the chest 12 of the patient 8 holding the handheld device 9 in his hands 10.

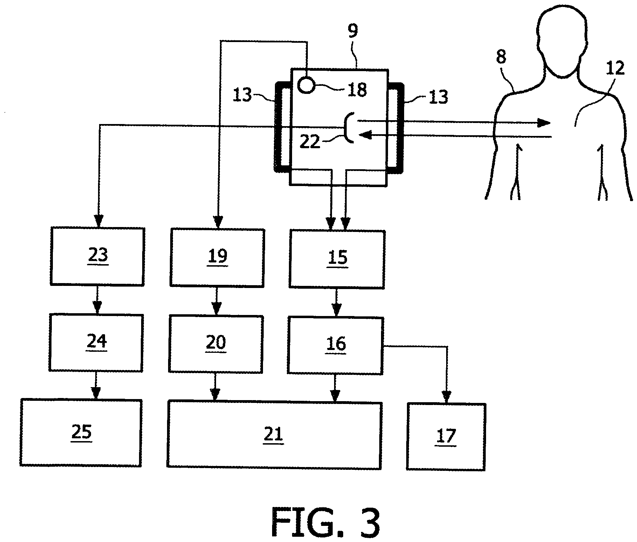

A block diagram of the system according to the invention is shown in Fig. 3. The handheld device 9 provides for three different measurements: heart rate, blood pressure and breathing activity. For that, the handheld device according to the invention is designed as follows:

For the heart rate measurement, the handheld device 9 comprises two electrodes which are formed by metal handles 13 which also serve for holding the handheld device. The handles 13 are connected to an ECG measuring unit comprising an ECG amplifier 15 and a peak detector 16. Then, the heart rate is calculated in a heart rate calculator 17.

For the blood pressure measurement, the handheld device 9 further comprises an optical sensor 18 for a photoplethysmography measurement which may be designed as described above. This optical sensor 18 is connected to a photoplethysmography measuring unit which comprises a photo amplifier 19 and a pulse detector 20. Then, the signal determined by the pulse detector 20 is output to a PAT (pulse arrival time) calculator 21 which also receives the signal output by the peak detector 16 of the ECG measuring unit. In the PAT calculator 21, the blood pressure is deduced from the PAT value and the ECG signal.

For the measurement of the breathing activities, the handheld device 9 is provided with a Doppler radar unit comprising an antenna 22 which emits electromagnetic waves towards the patient's chest 12 and receives electromagnetic waves reflected from the patient's chest 12. The signal received by the antenna 22 is fed to an RF front end 23 which is connected to a motion sensor 24. The signal output by the motion sensor 24 is then fed to a breathing rate calculator 25 for calculating the breathing rate of the patient 8.

In this way, an easy-to-use handheld solution for doing spot-checking of heart rate, blood pressure and breathing frequency is created. The solution is highly useful in hospital applications, in particular the so-called "spot-checking", when a nurse walks from patient bed to patient bed and wants to determine as quickly as possible vital parameters like heart rate, blood pressure and breathing rate.

At the moment, the nurse determines the patient's breathing rate by putting her hand onto the chest of the patient and looking at her wrist watch in order to see how many seconds a breathing cycle lasts. This method is rather inaccurate and bothersome for the nurse, so sometimes she just writes down an assumed figure. With the help of this preferred embodiment of the invention, these problems are overcome. The nurse simply gives the handheld device to the patient. He holds the device for a few seconds, during which his ECG, his pulse arrival time in the finger and his chest movement are measured with the help of the electrodes in the handles 13, the optical sensor 18 for the thumb and the Doppler radar, respectively.

From the ECG, it is easy to extract the heart rate. The pulse arrival time obtained with the help of the optical sensor is translated into blood pressure readings, and the Doppler radar measurement allows for determining the respiration rate. This way, all relevant parameters are captured with the help of a single, easy-to-use handheld device. The data can be stored directly on the handheld device 9 or transmitted through a wireless link, which is not shown in Fig. 3.

According to a preferred embodiment of the invention, the measurement of heart rate, blood pressure and respiration are used to give feedback to the patient 8 about his stress status. If combined with breathing instructions, a handheld device 9 for doing guided relaxation exercises is created.

Under resting conditions, the heart rate of healthy individuals exhibits a periodic variation. This rhythmic phenomenon, known as respiratory sinus arrhythmia (RSA), fluctuates with the phase of respiration: the heart rate increases during inspiration and decreases during expiration. This way the heart rate tends to synchronize with the patient's breathing activity under certain conditions.

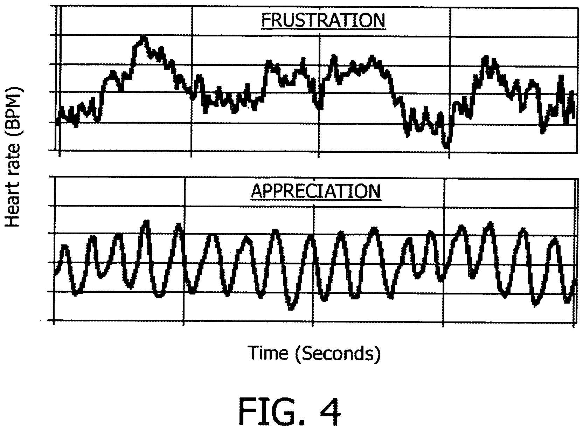

Fig. 4 shows how heart rate and respiration synchronize if a patient is in a positive or relaxed mood ("high coherence"), compared to the de-synchronization found if the patient is in a negative or stressed mood (low coherence). In the positive mood, the variation of the heart rate occurs in a sine wave manner. The third preferred embodiment of the invention allows to conduct simultaneously a measurement of heart rate variation and breathing activity, so the degree of coherence between the two can be calculated and used as a measure indicating the relaxation level of the patient. This can be done as follows:

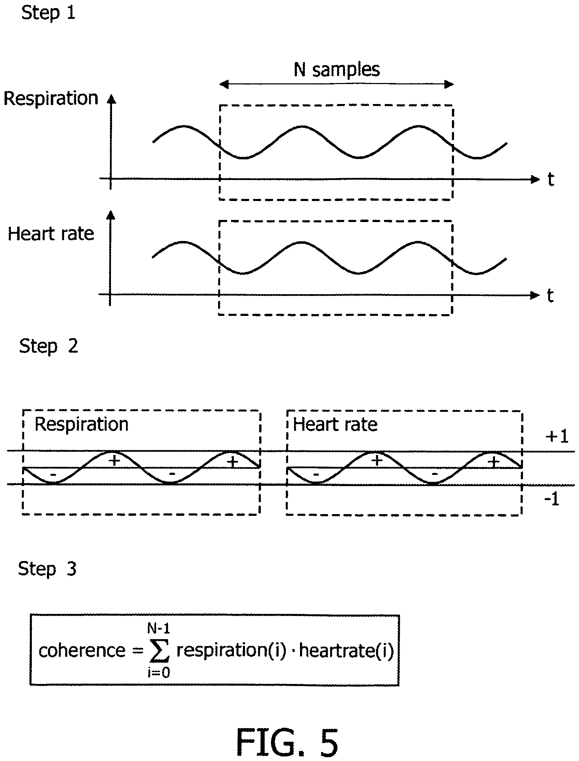

As shown in Fig. 5, in step 1, segments from the respiration rate signal and from the heart rate signal are cut from the original signals, both comprising N samples, respectively. Then, in step 2, the DC components from both segments are removed, and the amplitudes are normalized. Finally, in step 3, the coherence is calculated as the cross-correlation between the two segments:

$$coherence = \sum_{i=0}^{N-1} respiration(i) \cdot heartrate(i).$$

If the maxima in the respiratory signal coincide with the maxima in the heart rate signal, as it is shown in Fig. 5, the calculated degree of coherence is high, because positive values from the respiration segment are multiplied with positive values from the heart rate segment, and negative values from the respiration segment are multiplied with negative values from the heart rate segment. So in this case, all elements contributing to the sum calculation are positive. One can easily imagine that if maxima in the one segment coincide with minima in the other segment, the sum result is smaller in this case, because then positive values from the one segment are multiplied with negative values from the other segment, giving negative contributions to the sum calculation. Preferably, a guidance signal, indicating how the patient should breathe, is added to the system. The guidance signal can be adapted according to the relaxation status of the patient.

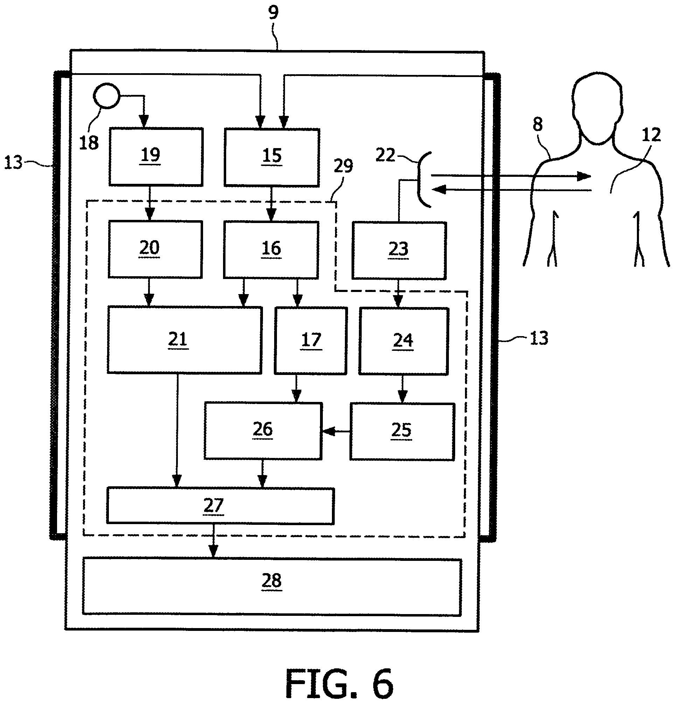

In Fig. 6 a block-diagram is depicted which shows a system according to a preferred embodiment: Additionally to the device shown in Fig. 3, according to this preferred embodiment of the invention a coherence calculator 26 is provided which is fed by the outputs of heart rate calculator 17 and breathing calculator 25. The ouput of coherence calculator 26 is then fed to a relaxation assessment unit 27 which also receives the output signal from PAT calculator 21. Finally an output device 28 like a display, a loudspeaker, an illumination or the like is provided for giving breathing instructions to the patient and/or for indicating the stress status.

The area 29 in Fig. 6 which is enclosed by a dashed line shows digital signal processing blocks that are preferably implemented on a microprocessor. As can be seen in Fig. 6, not only the degree of coherence between heart rate variation and breathing is taken into account in order to assess the relaxation level of the patient, but it is proposed to also use the blood pressure value determined with the help of the pulse arrival time of the pulse wave in the finger for this purpose.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope. Further, a patient is understood to be a human being or an animal which not necessarily has to be ill or diseased.

**Claims**

1.  A handheld device (9) for contactless respiration monitoring of a patient (8), comprising:

    a distance sensor for consecutively detecting the temporal distance variations relative to the patient's chest (12);
    a calculating unit (25) for determining the breathing activity based on the detected temporal distance variations;
    **characterised by**
    two handles (13) adapted for holding the device with both hands (10) of the patient (8) such that the distance sensor is directed to the patient's chest (12), and wherein the handles (13) comprise electrodes for an ECG measurement; and
    an optical sensor (18) for a photoplethysmography measurement provided on the device (9) such that, when holding the device (9), a finger (5) of the patient (8) automatically rests on the optical sensor (18).

2.  The device according to claim 1, wherein the distance sensor is based on emitting and receiving electromagnetic waves, and preferably comprises a Doppler radar sensor, preferably a two-channel Doppler radar sensor.

3.  The device according to any of claims 1 to 2, wherein the handks (13) are connected to an ECG measuring unit (15,16) provided in the device.

4.  The device according to any of claims 1 to 3, wherein the handheld device further comprises a heart rate calculator (17) for calculating the heart rate of the patient (8) from the ECG measurement.

5.  The device according to any of claims 1 to 4, wherein a photoplethysmography measuring unit (19, 20) which is connected to the optical sensor (18) is provided in the device and wherein the device is adapted for determining the blood pressure of the patient (8).

6.  The device according to claim 5 further comprising a pulse arrival time calculator (21) for calculating a pulse arrival time, wherein the pulse arrival time calculator (21) is adapted for receiving the signal determined by a pulse detector (20) of the photoplethysmography measuring unit (19, 20) and adapted for receiving the signal output by a peak detector (16) of the ECG measuring unit (15, 16) and adapted for deducing the blood pressure of the patient (8) from the calculated pulse arrival time value and the ECG measurement.

7.  The device according to claim 4, wherein the device further comprises an output unit (28) which is adapted for outputting a stress status indicator signal, based on coherence between the determined heart rate and the determined breathing activity.

8.  The device according to claim 5, wherein the device further comprises an output unit (28) which is adapted for outputting a stress status indicator signal, based on coherence between the determined heart rate and the determined breathing activity and also based on the blood pressure value of the patient (8).

9.  The device according to claim 7 or 8, wherein the output unit (28) further provides a guidance signal indicating how the patient (8) should breathe, wherein the guidance signal is automatically adapted according to the determined stress status of the patient (8).

10. The device according to any of claims 1 to 9, wherein the optical sensor (18) comprises a light unit (1) with a light emitter (2) for emitting light into tissue of a patient (8) and/or a light detector (3) for detecting a part of the emitted light after interaction with the tissue, wherein the light unit (1) is embedded in an elastic material (4).

11. The device according to claim 10, wherein the elastic material (4) is adapted for being contacted by the patient's finger (5).

12. The device according to claim 10 or 11, wherein the elasticity of the elastic material (4) lies in the range of typical elasticities of the tissue of a human finger.

13. The device according to any of claims 10 to 12, wherein the light unit (1) comprises an LED and a photodiode.

14. The device according to any of claims 10 to 13 wherein the elastic material (4) is not transparent for the light emitted by the light emitter (2).

**Patentansprüche**

1. Handgerät (9) zur kontaktlosen Atmungsüberwachung eines Patienten (8), das Folgendes umfasst:

   einen Abstandssensor zum aufeinanderfolgenden Detektieren der zeitlichen Abstandsveränderungen in Bezug auf die Brust (12) des Patienten;
   eine Berechungseinheit (25) zum Ermitteln der Atmungsaktivität basierend auf den detektierten zeitlichen Abstandsveränderungen;
   **gekennzeichnet durch**
   zwei Handgriffe (13), die vorgesehen sind, um das Gerät mit beiden Händen (10) des Patienten (8) derartig zu halten, dass der Abstandssensor auf die Brust (12) des Patienten ausgerichtet ist, und wobei die Handgriffe (13) Elektroden für eine EKG-Messung umfassen; und
   einen optischen Sensor (18) für eine Photoplethysmographie-Messung, der derartig an dem Gerät (9) vorgesehen ist, dass ein Finger (5) des Patienten (8) automatisch auf dem optischen Sensor (18) aufliegt, wenn das Gerät (9) festgehalten wird.

2. Gerät nach Anspruch 1, wobei der Abstandssensor auf dem Emittieren und Empfangen von elektromagnetischen Wellen basiert, und vorzugsweise einen Doppler-Radar-Sensor umfasst, vorzugsweise einen Zweikanal-Doppler-Radar-Sensor.

3. Gerät nach einem der Ansprüche 1 bis 2, wobei die Handgriffe (13) mit einer in dem Gerät vorgesehenen EKG-Messeinheit (15, 16) verbunden sind.

4. Gerät nach einem der Ansprüche 1 bis 3, wobei das Handgerät weiterhin eine Herzfrequenzberechnungseinheit (17) zum Berechnen der Herzfrequenz des Patienten (8) anhand der EKG-Messung umfasst.

5. Gerät nach einem der Ansprüche 1 bis 4, wobei eine Photoplethysmographie-Messeinheit (19, 20), die mit dem optischen Sensor (18) verbunden ist, in dem Gerät vorgesehen ist, und wobei das Gerät ausgelegt ist, um den Blutdruck des Patienten (8) zu ermitteln.

6. Gerät nach Anspruch 5, weiterhin mit einer Impulsankunftszeit-Berechnungseinheit (21) zum Berechnen einer Impulsankunftszeit, wobei die Impulsankunftszeit-Berechnungseinheit (21) vorgesehen ist, um das durch einen Impulsdetektor (20) der Photoplethysmographie-Messeinheit (19, 20) ermittelte Signal zu empfangen, und vorgesehen ist, um das durch einen Peak-Detektor (16) der EKG-Messeinheit (15, 16) ausgegebene Signal zu empfangen, und vorgesehen ist, um den Blutdruck des Patienten (8) von dem berechneten Impulsankunfszeitwert und der EKG-Messung abzuleiten.

7. Gerät nach Anspruch 4, wobei das Gerät weiterhin eine Ausgabeeinheit (28) umfasst, die vorgesehen ist, um ein Stressstatus-Indikatorsignal basierend auf der Kohärenz zwischen der ermittelten Herzfrequenz und der ermittelten Atmungsaktivität auszugeben.

8. Gerät nach Anspruch 5, wobei das Gerät weiterhin eine Ausgabeeinheit (28) umfasst, die vorgesehen ist, um ein Stressstatus-Indikatorsignal basierend auf der Kohärenz zwischen der ermittelten Herzfrequenz und der ermittelten Atmungsaktivität und auch basierend auf dem Blutdruckwert des Patienten (8) auszugeben.

9. Gerät nach Anspruch 7 oder 8, wobei die Ausgabeeinheit (28) weiterhin ein Führungssignal liefert, das angibt, wie der Patient (8) atmen sollte, wobei das Führungssignal automatisch entsprechend dem ermittelten Stressstatus des Patienten (8) angepasst wird.

**10.** Gerät nach einem der Ansprüche 1 bis 9, wobei der optische Sensor (18) eine Lichteinheit (1) mit einem Lichtemitter (2) zum Emittieren von Licht in Gewebe eines Patienten (8) und/oder einen Lichtdetektor (3) zum Detektieren eines Teils des emittierten Lichts nach der Wechselwirkung mit dem Gewebe umfasst, wobei die Lichteinheit (1) in ein elastisches Material (4) eingebettet ist.

**11.** Gerät nach Anspruch 10, wobei das elastische Material (4) vorgesehen ist, um durch den Finger (5) des Patienten berührt zu werden.

**12.** Gerät nach Anspruch 10 oder 11, wobei die Elastizität des elastischen Materials (4) in dem Bereich von typischen Elastizitäten des Gewebes eines menschlichen Fingers liegt.

**13.** Gerät nach einem der Ansprüche 10 bis 12, wobei die Lichteinheit (1) eine LED und eine Fotodiode umfasst.

**14.** Gerät nach einem der Ansprüche 10 bis 13, wobei das elastische Material (4) nicht für das durch den Lichtemitter (2) emittierte Licht transparent ist.

**Revendications**

**1.** Dispositif de poche (9) pour une surveillance respiratoire (57) d'un patient (8), comprenant :

un capteur de distance permettant de détecter consécutivement des variations de distance temporelles par rapport à la poitrine (12) du patient ;
une unité de calcul (25) permettant de déterminer l'activité respiratoire d'après les variations de distance temporelles détectées ;
**caractérisé par**
deux poignées (13) adaptées pour tenir le dispositif avec les deux mains (10) du patient (8) de sorte que le capteur de distance est dirigé vers la poitrine (12) du patient, et dans lequel les poignées (13) comprennent des électrodes pour une mesure ECG (électrocardiogramme) ; et
un capteur optique (18) pour une mesure de pléthysmographie optique disposé sur le dispositif (9) de sorte que, lorsqu'il tient le dispositif (9), le patient (8) a le doigt (5) qui repose automatiquement sur le capteur optique (18).

**2.** Dispositif selon la revendication 1, dans lequel le capteur de distance se base sur des ondes électromagnétiques d'émission et de réception, et comprend de préférence un capteur radar à effet Doppler, de préférence un capteur radar à effet Doppler bicanal.

**3.** Dispositif selon l'une quelconque des revendications 1 à 2, dans lequel les poignées (13) sont connectées à une unité de mesure ECG (15, 16) pourvue dans le dispositif.

**4.** Dispositif selon l'une quelconque des revendications 1 à 3, dans lequel le dispositif de poche comprend en outre un calculateur de rythme cardiaque (17) permettant de calculer le rythme cardiaque du patient (8) à partir de la mesure ECG.

**5.** Dispositif selon l'une quelconque des revendications 1 à 4, dans lequel une unité de mesure de pléthysmographie optique (19, 20) qui est raccordée au capteur optique (18) est pourvue dans le dispositif et dans lequel le dispositif est adapté pour déterminer la tension artérielle du patient (8).

**6.** Dispositif selon la revendication 5, comprenant en outre un calculateur de temps d'arrivée d'impulsion (21) permettant de calculer un temps d'arrivée d'impulsion, dans lequel le calculateur de temps d'arrivée d'impulsion (21) est adapté pour recevoir le signal déterminé par un détecteur d'impulsion (20) de l'unité de mesure de pléthysmographie optique (19, 20) et adapté pour recevoir le signal fourni en sortie par un détecteur de crête (16) de l'unité de mesure ECG (15, 15) et adapté pour déduire la tension artérielle du patient (8) d'après la valeur de temps d'arrivée d'impulsion calculée et la mesure ECG.

**7.** Dispositif selon la revendication 4, dans lequel le dispositif comprend en outre une unité de sortie (28) qui est adaptée pour fournir en sortie un signal indicateur de statut de stress, d'après une cohérence entre le rythme cardiaque déterminé et l'activité respiratoire déterminée.

8. Dispositif selon la revendication 5, dans lequel le dispositif comprend en outre une unité de sortie (28) qui est adaptée pour fournir en sortie un signal indicateur de statut de stress, d'après une cohérence entre le rythme cardiaque déterminé et l'activité respiratoire déterminée et d'après également la valeur de tension artérielle du patient (8).

9. Dispositif selon la revendication selon la revendication 7 ou 8, dans lequel l'unité de sortie (28) fournit en outre un signal de guidance indiquant comment le patient (8) devrait respirer, dans lequel le signal de guidance est adapté automatiquement selon le statut de stress déterminé du patient (8).

10. Dispositif selon l'une quelconque des revendications 1 à 9, dans lequel le capteur optique (18) comprend une unité de lumière (1) avec un émetteur de lumière (2) permettant d'émettre de la lumière dans le tissu d'un patient (8) et/ou un détecteur de lumière (3) permettant de détecter une partie de la lumière émise après interaction avec le tissu, dans lequel l'unité de lumière (1) est incorporée dans un matériau élastique (4).

11. Dispositif selon la revendication 10, dans lequel le matériau élastique (4) est adapté pour être touché par le doigt (5) du patient.

12. Dispositif selon la revendication 10 ou 11, dans lequel l'élasticité du matériau élastique (4) réside dans la gamme d'élasticités typiques du tissu d'un doigt humain.

13. Dispositif selon l'une quelconque des revendications 10 à 12, dans lequel l'unité de lumière (1) comprend une DEL et une photodiode.

14. Dispositif selon l'une quelconque des revendications 10 à 13, dans lequel le matériau élastique (4) n'est pas transparent pour la lumière émise par l'émetteur de lumière (2).

FIG. 1a

FIG. 1b

FIG. 2a

FIG. 2b

FIG. 2c

FIG. 3

FIG. 4

Step 1

N samples

Respiration

t

Heart rate

t

Step 2

Respiration    Heart rate    +1

+    +    +    +

-    -    -    -

-1

Step 3

$$coherence = \sum_{i=0}^{N-1} respiration(i) \cdot heartrate(i)$$

# FIG. 5

15

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080077015 A1
- WO 2008027509 A2
- US 20050288601 A1